# FASCICULE DE BREVET EUROPEEN

(11) **EP 2 339 977 B1**
(45) Date de publication et mention de la délivrance du brevet: **03.05.2017**
(21) Numéro de dépôt: 09741356.1
(22) Date de dépôt: 02.09.2009
(51) Int. Cl.: A61B 17/72

(54) **IMPLANT INTRAMEDULLAIRE RESORBABLE ENTRE DEUX OS OU DEUX FRAGMENTS OSSEUX**
RESORPTIVES INTRAMEDULLÄRES IMPLANTAT ZWISCHEN ZWEI KNOCHEN ODER ZWEI KNOCHENFRAGMENTEN
RESORPTIVE INTRAMEDULLARY IMPLANT BETWEEN TWO BONES OR TWO BONE FRAGMENTS

(30) Priorité: 09.09.2008 FR 0856035
(43) Date de publication de la demande: 06.07.2011
(73) Titulaire: Stryker European Holdings I, LLC, Kalamazoo, MI 49002 (US)
(72) Inventeur: PRANDI, Bernard, F-35700 Rennes (FR); AUGOYARD, Marc, F-69160 Tassin La Demi-Lune (FR); LEDERMANN, Thomas, CH-8733 Eschenbach (CH); MEUSNIER, Tristan, F-42100 Saint-Etienne (FR); PEYROT, Jacques, F-69160 Tassin La Demi Lune (FR); FELLMANN, Judith, CH-8712 Stafa (CH)
(74) Mandataire: Regimbeau
(86) Numéro de dépôt international: PCT/FR2009/051658
(87) Numéro de publication internationale: WO 2010/029246

(56) Documents cités:
- WO-A-2006/109004
- FR-A- 2 846 545
- GB-A- 2 430 625
- US-B1- 7 041 106

## Description

L'invention se rattache au secteur technique des implants orthopédiques, notamment pour arthrodèse et ostéosynthèse.

Plus particulièrement, l'invention concerne un implant intramédullaire pour réaliser une arthrodèse entre deux parties d'os ou une ostéosynthèse entre deux fragments osseux, notamment dans le cas de la main ou du pied.

Différentes solutions ont été proposées pour réaliser ces fonctions.

Par exemple, une solution ressort de l'enseignement de la demande de brevet FR 2.884.406 dont le demandeur de la présente est également titulaire. Ce brevet décrit un dispositif d'ostéosynthèse intramédullaire constitué par un corps de forme allongée dont les extrémités constituent des zones d'ancrage coopérant par les parties d'os à immobiliser. Les zones d'ancrage sont profilées et réalisées dans un matériau sélectionné pour permettre l'introduction dans les parties d'os, puis assurer un ancrage dans lesdites parties d'os en évitant tout mouvement de rotation en résistant à la traction et en maintenant un effort de compression.

Une autre solution ressort également de la demande de brevet FR 07.02003 appartenant toujours au demandeur. Ce document décrit un implant sous la forme de deux zones d'ancrage reliées par une zone centrale et dont la forme générale est sensiblement inscrite dans un rectangle très allongé en présentant une forme sensiblement en X, de manière à constituer, au niveau des zones d'ancrage, deux pattes aptes à s'écarter par effet élastique ou par effet mémoire de formes.

A partir de cette conception, différents critères ont été établis pour rendre l'implant facile à poser et efficace afin de générer une stabilité primaire et secondaire du foyer d'ostéosynthèse ou d'arthrodèse.

Toutefois, ces solutions ne sont pas adaptées dans le cas d'un implant en matériau résorbable.

A partir de cet état de la technique, le problème que se propose de résoudre l'invention est d'améliorer encore l'ancrage et la stabilité de l'implant et son adaptation à la morphologie du site d'implantation, lorsque ledit implant est réalisé dans un matériau résorbable.

Pour résoudre un tel problème, il a été conçu et mis au point implant intramédullaire résorbable entre deux os ou deux fragments osseux, constitué, d'une manière connue, par un corps monobloc de forme générale allongée présentant, à chaque extrémité, des zones d'ancrage avec les parties d'os considérées. Selon l'invention, l'une des zones est de section cylindrique, tandis que l'autre zone est de section méplate.

Avantageusement, l'implant est réalisé dans un matériau résorbable dont les propriétés mécaniques sont déterminées pour durer le temps de la consolidation nécessaire, de sorte que ledit implant est résorbable au-delà de 6 mois. Par exemple, l'implant est composé de polymère ou copolymère d'acide lactique (PLA, PGA, ...)

Compte tenu des caractéristiques mécaniques spécifiques des matériaux résorbables, et pour résoudre le problème posé d'améliorer l'ancrage et la stabilité, la zone de section cylindrique est filetée et présente une conicité de section dégressive en direction de son extrémité libre.

Pour résoudre le problème posé de permettre une déformation par élasticité, en générant ainsi une expansion adaptée à la géométrie du site et à la propriété du matériau, la zone de section méplate présente, sensiblement da sa partie médiane, une ouverture apte à permettre une déformation par élasticité de ladite zone. L'ouverture délimite au moins deux pattes d'ancrage.

Il apparaît donc que la combinaison d'une zone d'ancrage cylindrique et filetée et d'une zone d'ancrage de section méplate, est particulièrement avantageuse compte tenu du problème posé à résoudre.

Pour résoudre le problème posé de résister aux contraintes de cisaillement et de flexion susceptibles de s'exercer au niveau du foyer osseux, entre les deux zones d'ancrage, le corps présente une zone centrale de transition apte à résister aux contraintes de cisaillement et de flexion s'exerçant au niveau du foyer osseux et apte à faire office de butée.

A partir de cette conception de base de l'implant, soit les zones d'ancrage sont disposées en alignement coaxial, soit les zones d'ancrage sont décalées angulairement entre 1° et 30° environ, et, avantageusement, de 10°. La ligne de pliage entre les zones d'ancrage est située pour correspondre sensiblement à une ligne d'arthrodèse des os considérés.

L'invention est exposée ci-après plus en détail à l'aide des figures des dessins annexés dans lesquels :
- la figure 1 est une vue en perspective de l'implant ;

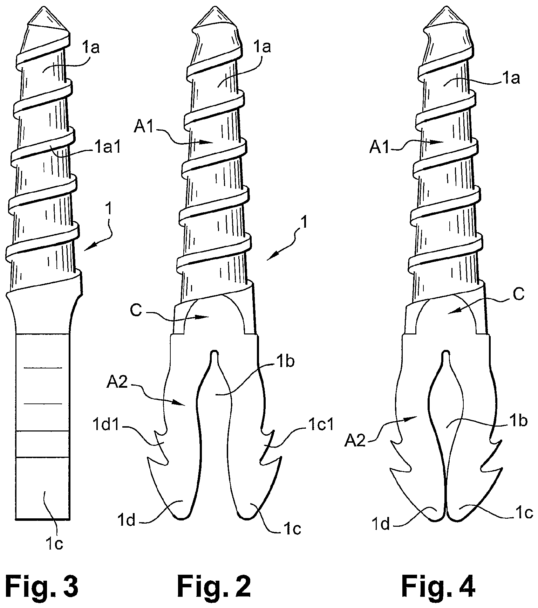

- la figure 2 est une vue de face de l'implant avant introduction dans la partie d'os considérée ;
- la figure 3 est une vue de côté correspondant à la figure 2 ;
- la figure 4 est une vue semblable à la figure 2 montrant le positionnement des pattes d'ancrage de la section méplate après introduction ;
- la figure 5 est une vue en perspective d'une autre forme de réalisation avantageuse de l'implant ;
- les figures 6 et 7 montrent la mise en place de l'implant dans deux parties d'os.

L'implant, conforme à l'invention, comprend un corps (1) monobloc de forme allongée et présentant une première zone dite proximale (A1) et une seconde zone dite distale (A2). L'ensemble du corps de l'implant est réalisé dans un matériau résorbable dont les propriétés mécaniques sont déterminées pour que l'implant se résorbe dans un délai supérieur à 6 mois environ. Dans un exemple de réalisation, l'implant est composé de polymère ou copolymère d'acide lactique (PLA, PGA, ...).

Comme il sera indiqué dans la suite de la description, les zones (A1) et (A2) présentent des agencements d'ancrage avec les parties d'os correspondantes. En considérant les caractéristiques spécifiques du matériau résorbable, et pour résoudre le problème posé de l'ancrage et de la stabilité, la zone (A1) est de section cylindrique, tandis que l'autre zone (A2) est de section méplate.

La zone (A1) est constituée par une portée cylindrique (la) présentant une conicité réduite en direction de l'extrémité libre. La portée (la) présente une nervure hélicoïdale faisant office de filetage (1a1).

La zone (A2) de section méplate présente, sensiblement dans sa partie médiane, une ouverture (1b) apte à permettre une déformation par élasticité de ladite zone (A2). Plus particulièrement, l'ouverture (1b) délimite au moins deux pattes d'ancrage (1c) et (1d) présentant, chacune, au moins un cran externe (1c1), (1d1).

Avantageusement, entre les deux zones (A1) et (A2), le corps (1) présente une zone centrale (C) de transition apte à résister aux contraintes de cisaillement et de flexion susceptibles de s'exercer au niveau du foyer osseux. A titre indicatif, nullement limitatif, cette zone médiane (C) peut avoir une largeur de 3,5 mm environ pour une épaisseur de 2 mm environ, pour une longueur d'implant comprise entre 15 et 25 mm environ avec un diamètre de 2 ou 3 mm environ, au niveau de la zone (A1).

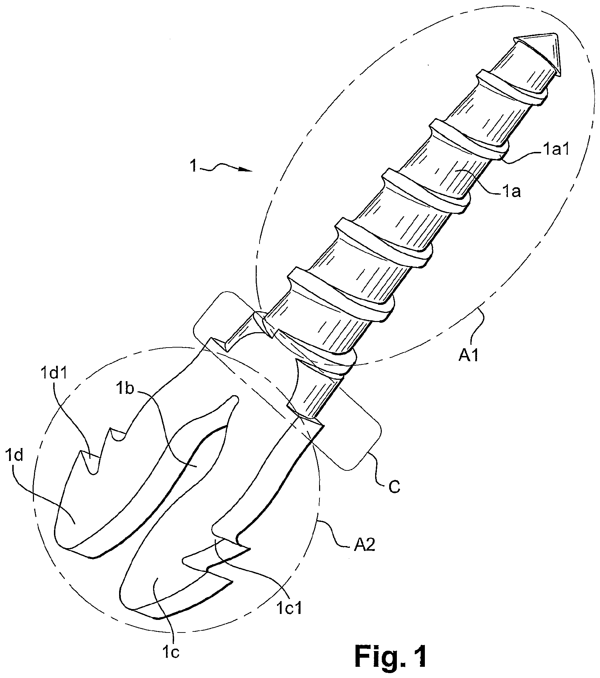

Dans la forme de réalisation illustrée figure 1, les deux zones (A1) et (A2) sont disposées en alignement coaxial.

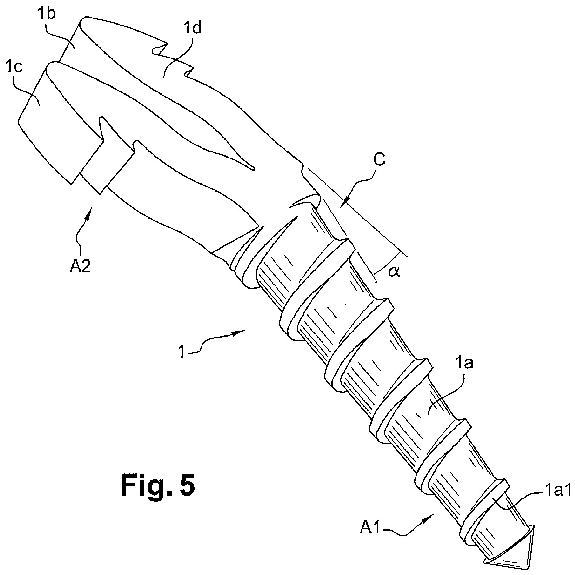

Pour répondre au problème de l'adaptation à la morphologie du site d'implantation, les zones d'ancrage (A1) et (A2) peuvent être décalées angulairement d'un angle α adaptée à la géométrie du site osseux. Cet angle α est compris entre 1° et 30° environ et, avantageusement, de l'ordre de 10° dans le cas d'une application de l'implant à une arthrodèse du pied (figure 5).

Dans cette forme de réalisation où les deux zones d'ancrage sont décalées angulairement, la ligne de pliage est située pour correspondre sensiblement à une ligne d'arthrodèse des parties d'os considérées.

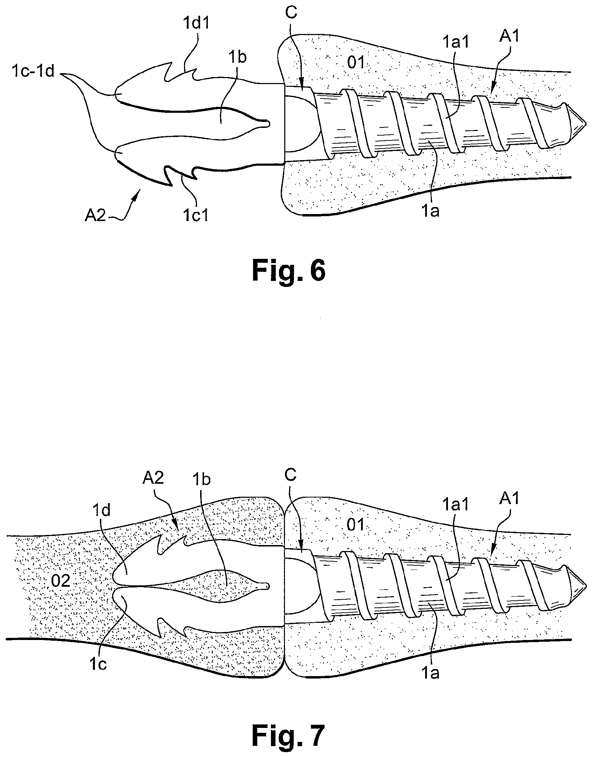

On renvoie aux figures 6 et 7 qui montrent, schématiquement, le positionnement de l'implant selon l'invention entre deux parties d'os (O1) et (O2). Après réalisation des logements osseux adaptés, au moyen d'un instrument du type râpe, l'opérateur visse la portée filetée (la) dans la partie d'os considérée (O1) jusqu'à, sensiblement la zone médiane (C) qui fait office de butée empêchant l'enfoncement trop important de l'implant dans l'os considéré (figure 6). L'opérateur vient ensuite rechausser la deuxième partie d'os (O2) sur les pattes d'ancrage (1d) et (1c) de la zone (A2), lesquelles se resserrent par élasticité (figure 7).

Ainsi, la technique opératoire peut être la suivante :
- Forage des deux trous avec un foret adapté classique ;
- Préparation des logements avec une râpe pour le côté méplat et un taraud pour préparer le pas de vis intérieur côté cylindrique ;
- Utilisation d'un tournevis avec embout préhensif ;
- Vissage côté cylindrique, en général P1, pour une arthrodèse IPP du pied ;
- Rechaussage de l'os côté méplat sur l'implant.

Les avantages ressortent bien de la description, en particulier on souligne et on rappelle que la combinaison des deux zones d'ancrage (A1) et (A2), respectivement de section cylindrique et de section méplate, améliore, d'une manière significative, l'ancrage et la stabilité de l'implant adapté à la géométrie du foyer osseux et aux propriétés du matériau, à savoir un matériau résorbable.

## Revendications

1. Implant intramédullaire résorbable pour réaliser une arthrodèse entre deux parties d'os ou une ostéosynthèse entre deux fragments osseux, constitué par un corps monobloc (1) de forme générale allongée présentant, à chaque extrémité, des zones d'ancrage avec lesdites parties d'os ou lesdits fragments osseux considérés, **caractérisé en ce que** l'une des zones (A1) est de section cylindrique, tandis que l'autre zone (A2) est de section méplate.

2. Implant selon la revendication 1, **caractérisé en ce que** la zone (A1) de section cylindrique est filetée.

3. Implant selon l'une des revendications précédentes, **caractérisé en ce que** la zone (A1) comporte une extrémité libre et présente une conicité de section dégressive en direction de son extrémité libre.

4. Implant selon l'une quelconque des revendications précédentes, **caractérisé en ce que** la zone (A2) de section méplate présente, sensiblement dans sa partie médiane, une ouverture (1 b) apte à permettre une déformation par élasticité de ladite zone.

5. Implant selon la revendication 4, **caractérisé en ce que** l'ouverture (1 b) délimite au moins deux pattes d'ancrage (1c) et (1d).

6. Implant selon l'une quelconque des revendications 1 à 5 , **caractérisé en ce que**, entre les deux zones d'ancrage (A1) et (A2), le corps présente une zone centrale (C) de transition apte à résister aux contraintes de cisaillement et de flexion s'exerçant au niveau du foyer osseux et apte à faire office de butée.

7. Implant selon l'une quelconque des revendications 1 à 6, **caractérisé en ce que** les zones (A1) et (A2) d'ancrage sont disposées en alignement coaxial.

8. Implant selon l'une quelconque des revendications 1 à 6, **caractérisé en ce que** les zones (A1) et (A2) d'ancrage sont décalées angulairement.

9. Implant selon la revendication 8 **caractérisé en ce que** les zones d'ancrage (A1) et (A2) sont décalées angulairement entre 1[deg.] et 30[deg.] environ, et, avantageusement, de 10[deg.].

10. Implant selon l'une des revendications 8 et 9, **caractérisé en ce que** la ligne de pliage, entre les zones d'ancrage, est située pour correspondre sensiblement à une ligne d'arthrodèse des os considérés.

11. Implant selon l'une des revendications 1 à 10, **caractérisé en ce que** l'implant est apte à être déformé par élasticité.

## Patentansprüche

1. Resorbierbares intramedulläres Implantat zur Durchführung einer Arthrodese zwischen zwei Knochenteilen oder einer Osteosynthese zwischen zwei Knochenfragmenten, gebildet von einem allgemein länglichen Einblockkörper (1), der an jedem Ende Verankerungszonen mit den entsprechenden Knochenteilen oder Knochenfragmenten aufweist, **dadurch gekennzeichnet, dass** eine der Zonen (A1) einen zylindrischen Querschnitt hat, wogegen die andere Zone (A2) abgeflachten Querschnitts ist.

2. Implantat nach Anspruch 1, **dadurch gekennzeichnet, dass** die Zone (A1) zylindrischen Querschnitts gewindet ist.

3. Implantat nach einem der vorangehenden Ansprüche, **dadurch gekennzeichnet, dass** die Zone (A1) ein freies Ende aufweist und eine Konizität mit abnehmendem Querschnitt in Richtung ihres freien Endes aufweist.

4. Implantat nach einem der vorangehenden Ansprüche, **dadurch gekennzeichnet, dass** die Zone (A2) mit abgeflachtem Querschnitt etwa in ihrem mittleren Teil eine Öffnung (1b) aufweist, die imstande ist, eine Verformung durch Elastizität der Zone zu erlauben.

5. Implantat nach Anspruch 4, **dadurch gekennzeichnet, dass** die Öffnung (1b) mindestens zwei Verankerungsfüße (1c) und (1d) begrenzt.

6. Implantat nach einem der Ansprüche 1 bis 5, **dadurch gekennzeichnet, dass** der Körper zwischen den zwei Verankerungszonen (A1) und (A2) eine zentrale Übergangszone (C) aufweist, die imstande ist, Scher- und Flexionskräften zu widerstehen, die im Bereich des Knochenmaterials wirken, und imstande ist, als Anschlag zu dienen.

7. Implantat nach einem der Ansprüche 1 bis 6, **dadurch gekennzeichnet, dass** die Verankerungszonen (A1) und (A2) koaxial fluchtend angeordnet sind.

8. Implantat nach einem der Ansprüche 1 bis 6, **dadurch gekennzeichnet, dass** die Verankerungszonen (A1) und (A2) winklig versetzt sind.

9. Implantat nach Anspruch 8, **dadurch gekennzeichnet, dass** die Verankerungszonen (A1) und (A2) zwischen zirka 1° und 30° und in vorteilhafter Weise um 10° winklig versetzt sind.

10. Implantat nach einem der Ansprüche 8 und 9, **dadurch gekennzeichnet, dass** die Faltlinie zwischen den Verankerungszonen angeordnet ist, um etwa einer Arthrodeselinie der betroffenen Knochen zu entsprechen.

11. Implantat nach einem der Ansprüche 1 bis 10, **dadurch gekennzeichnet, dass** das Implantat imstande ist, elastisch verformt zu sein.

## Claims

1. A resorbable intramedullary implant for carrying out anarthrodesis between two bone parts or osteosynthesis between two bone fragments, formed of a one-piece body (1) having a general elongated shape with, at each end, zones for anchoring with said respective bone parts or said bone fragments, **characterized in that** one of the zones (A1) is of cylindrical section, whereas the other zone (A2) is of flat section.

2. The implant according to claim 1, **characterized in that** the zone (A1) of cylindrical section is threaded.

3. The implant according to one of the preceding claims, **characterized in that** the zone (A1) includes a free end and has a conicity of decreasing section toward its free end.

4. The implant according to any one of the preceding claims, **characterized in that** the zone (A2) of flat section has substantially in its median portion an opening (1b) adapted to allow deformation by elasticity of said zone.

5. The implant according to claim 4, **characterized in that** the opening (1b) defines at least two anchoring legs (1c) and (1d)

6. The implant according to any of claims 1 to 5, **characterized in that**, between the two anchor zones (A1) and (A2), the body has a central transition zone (C) adapted to resist shear and flexion forces occurring at the bone site and adapted to serve as an abutment.

7. The implant according to any one of claims 1 to 6, **characterized in that** the anchor zones (A1) and (A2) are disposed in coaxial alignment.

8. The implant according to any one of claims 1 to 6, **characterized in that** the anchor zones (A1) and (A2) are angularly offset.

9. The implant according to claim 8, **characterized in that** the anchor zones (A1) and (A2) are angularly offset between about 1° and 30°, and, advantageously, by 10°.

10. The implant according to one of claims 8 and 9, **characterized in that** the line of bending, between the anchoring zones, is located so as to correspond substantially to a line for arthrodesis of the bones being considered.

11. The implant according to one of claims 1 to 10, **characterized in that** the implant is adapted to be elastically deformed.
